(19) Europäisches Patentamt
European Patent Office
Office européen des brevets

(11) Numéro de publication: **0 053 950**
**A1**

(12) **DEMANDE DE BREVET EUROPEEN**

(21) Numéro de dépôt: 81401625.9

(22) Date de dépôt: 16.10.81

(51) Int. Cl.³: C 07 D 495/04
//A61K31/435, (C07D495/04, 333/00, 221/00)

(30) Priorité: 28.11.80 FR 8025276

(43) Date de publication de la demande: 16.06.82 Bulletin 82/24

(84) Etats contractants désignés: AT BE CH DE GB IT LI LU NL SE

(71) Demandeur: SANOFI, Société dite:
40, Avenue George V
F-75008 Paris(FR)

(72) Inventeur: Boigegrain, Robert
28 Ancienne Route Impériale
F-31120 Portet-sur-Garonne(FR)

(72) Inventeur: Maffrand, Jean-Pierre
5 Rue du Corps-Franc Pommiès
F-31120 Portet-sur-Garonne(FR)

(72) Inventeur: Suzuki, Norio
942-116 Naganumahara-cho
Chiba-Shi Chiba(JP)

(72) Inventeur: Matsubayashi, Kiuichi
21-22 Yagigaya-cho Funabashi-Shi
Chiba(JP)

(72) Inventeur: Ashida, Shinichiro
1-12 Katemama, 2 Chome Ichikawa-Shi
Chiba(JP)

(74) Mandataire: Bressand, Georges et al,
c/o CABINET LAVOIX 2 Place d'Estienne d'Orves
F-75441 Paris Cedex 09(FR)

(54) **Nouveau procédé de préparation de dérivés de la tétrahydro-5,6,7,7a-4H-thiéno(3,2-c)pyridinone-2.**

(57) La présente invention est relative à un procédé de préparation de dérivés de la tétrahydro-5,6,7,7a 4H-thiéno(3,2-c)pyridinone-2 de formule:

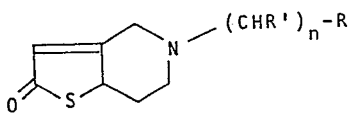

(I) :

dans laquelle R représente l'hydrogène ou un radical phényle éventuellement substitué par au moins un atome d'halogène ou un groupe alcoyle inférieur, alcoxy inférieur, nitro, carboxy, alcoxycarbonyle ou cyano; R' représente l'hydrogène, un groupe alcoyle inférieur; et n est zéro ou un nombre entier de 1 à 4, ainsi que leurs sels d'addition avec des acides minéraux ou organiques, caractérisé en ce que :

a) on traite par une solution d'eau oxygénée, un composé de formule II suivante :

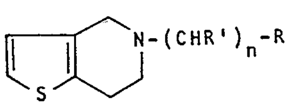

(II),

dans laquelle R, R' et n sont tels que définis ci-dessus, en solution dans un mélange acide acétique/acide bromhydrique;

b) on transforme en organomagnésien, dans un solvant inerte anhydre, le dérivé bromé obtenu à l'issue de l'étape a) et ayant la formule III suivante :

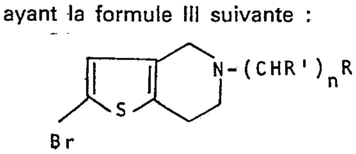

(III) ;

puis on condense le magnésien obtenu avec du perbenzoate de t-butyle pour obtenir le composé de formule IV suivante :

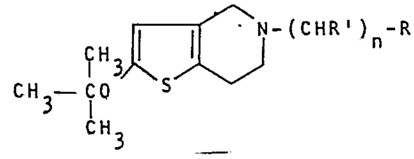

(IV); et

c) on transforme le composé de formule IV obtenu ci-dessus en composé de formule I par chauffage à des températures comprises entre 80 et 180°C, en présence d'un acide minéral ou organique.

EP 0 053 950 A1

0053950

Nouveau procédé de préparation de dérivés de la tétrahydro-5,6,7,7a 4H-thiéno(3,2-c)pyridinone-2.-

La présente invention est relative à un nouveau procédé de préparation de tétrahydro-5,6,7,7a 4H-thiéno(3,2-c)pyridinones-2 de formule :

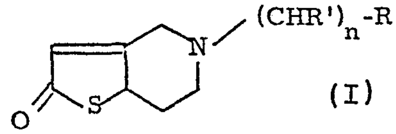

(I)

dans laquelle R représente l'hydrogène ou un radical phényle éventuellement substitué par au moins un atome d'halogène ou un groupe alcoyle inférieur, alcoxy inférieur, nitro, carboxy, alcoxycarbonyle ou cyano; R' représente l'hydrogène, un groupe alcoyle inférieur; et n est zéro ou un nombre entier de 1 à 4, ainsi que leurs sels d'addition avec des acides minéraux ou organiques.

Ces composés qui présentent des propriétés antiagrégantes plaquettaires et anti-thrombotiques font l'objet d'une autre demande de brevet déposée ce jour par la demanderesse.

Ils sont par ailleurs inclus dans une formule générale revendiquée dans les brevets français N° 73 03 503 et N° 75 24 486 sous leur forme tautomère suivante

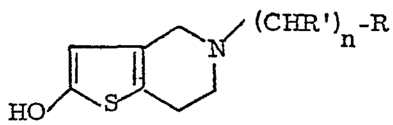

Cependant, aucun de ces produits n'est spécifiquement décrit.

On connaît d'après l'article de J.Am.Chem. Soc, vol. 72, décembre 1950, p. 5543-46, un procédé de préparation du thiénol-2 à partir de bromo-2 thiophène, par l'intermédiaire d'un magnésien. Dans ce procédé le magnésien est transformé en alcool par oxydation à l'aide d'oxygène en présence de bromure d'isopropyl magnésium dans la solution de magnésien, afin d'éviter la formation de produits secondaires indésirables.

La présente invention a ainsi pour objet un procédé de préparation des composés de formule I décrite ci-dessus, caractérisé en ce que :

a) on traite par une solution d'eau oxygénée un composé de formule II suivante :

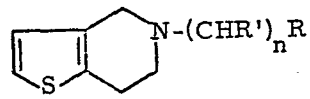

(II),

dans laquelle R, R' et n sont tels que définis ci-dessus, en solution dans un mélange acide acétique/acide bromhydrique;

b) on transforme en organomagnésien, dans un solvant inerte anhydre, le dérivé bromé obtenu à l'issue de l'étape a) et ayant la formule III suivante :

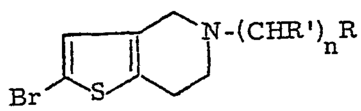

(III) ;

puis on condense le magnésien obtenu avec du perbenzoate de t-butyle pour obtenir le composé de formule IV suivante

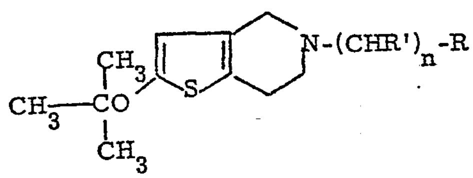

(IV) ; et

c) on transforme le composé de formule IV obtenu ci-dessus en composé de formule I par chauffage à des températures comprises entre 80 et 180°C, en présence d'un acide minéral ou organique.

Le procédé de la présente invention permet donc d'éviter l'oxydation du magnésien intermédiaire par l'oxygène, décrit dans la technique antérieure.

Le mélange d'acide acétique/acide bromhydrique est compris de préférence dans les limites suivantes : de 50/50 à 80/20.

L'acide bromhydrique utilisé au cours de l'étape a) est de préférence de l'acide à 48% et l'acide acétique est de préférence pur.

L'eau oxygénée est utilisée sous forme d'une solution alcoolique aqueuse telle qu'une solution méthanolique d'eau oxy-

génée à 30 %.

Au cours de l'étape b), on utilise un solvant inerte tel que le tétrahydrofuranne à la fois pour préparer le composé organomagnésien et pour la condensation ultérieure avec le perbenzoate de t-butyle, du composé magnésien obtenu.

Le dérivé organomagnésien est préparé de façon classique, par exemple à l'aide de magnésium métallique et d'un halogénure d'alcoyle tel que le bromure d'isopropyle, en milieu anhydre.

Le magnésien n'est pas isolé du milieu avant sa condensation avec le perbenzoate de t-butyle.

Au cours de l'étape c), on ajoute un acide minéral ou organique tel que, par exemple, l'acide p.toluènesulfonique.

Le procédé selon la présente invention est représenté par le schéma réactionnel suivant :

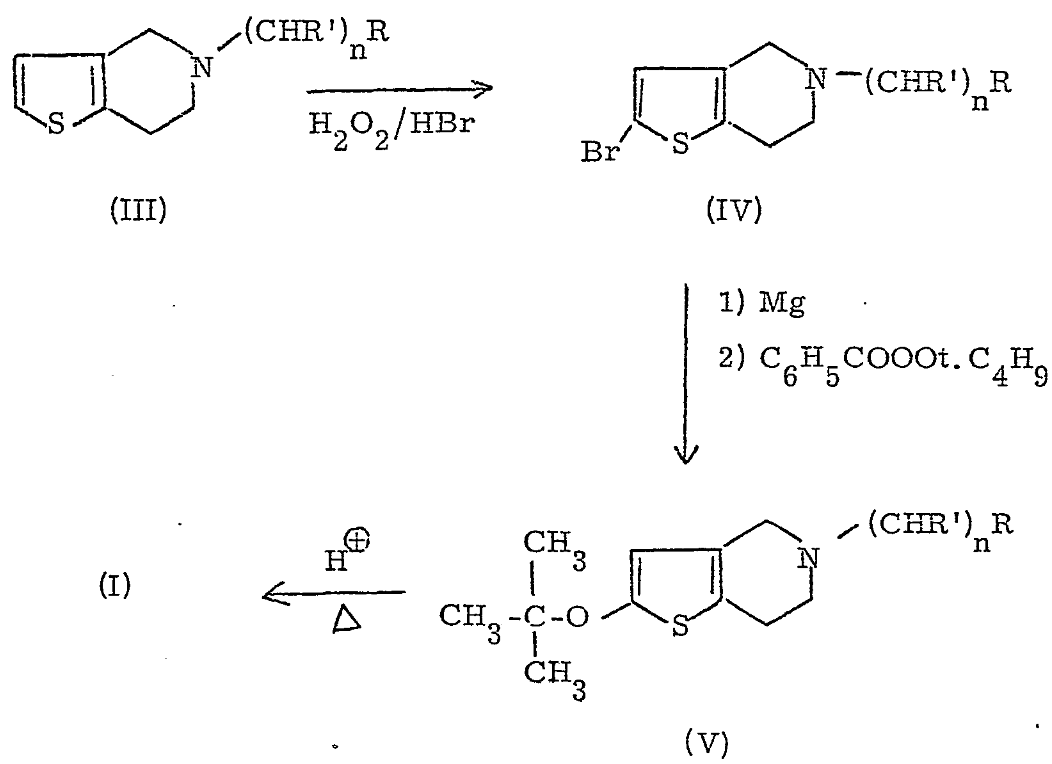


(III) (IV) (V) (I)

Les produits de départ de formule (II) ont été préparés selon des procédés décrits dans la littérature, par exemple selon les procédés décrits dans les deux brevets déjà cités, à savoir Fr 73 03 503 et Fr 75 24 486.

Les exemples suivants illustrent l'invention :

EXEMPLE 1 : (Chloro-2 benzyl)-5 tétrahydro-5, 6, 7, 7a 4H-thiéno (3, 2-c) pyridinone-2, de formule (I)

($R = 2\text{-}Cl\text{-}C_6H_4$ ; R' = H ; n = 1) dérivé n° 1

On ajoute, goutte à goutte, un mélange d'eau oxygénée à 30 % et de méthanol (3 $cm^3$/10 $cm^3$) à une solution refroidie à 0°C de 2, 63 g de (chloro-2 benzyl)-5 tétrahydro-4, 5, 6, 7 thiéno (3, 2-c) pyridine dans 10 $cm^3$ d'acide acétique et 6 $cm^3$ d'acide bromhydrique à 48 %.

L'addition terminée, on agite 10 mn à température ambiante, on ajoute une solution d'hydrosulfite de sodium puis une solution de soude et extrait avec un mélange benzène-chloroforme. Les extraits organiques sont lavés à l'eau, séchés sur sulfate de sodium et filtrés. On ajoute au filtrat une solution de gaz chlorhydrique dans le méthanol et on concentre sous vide. La recristallisation du résidu solide, dans l'éthanol fournit 2, 0 g de cristaux beiges, de forme plate, de chlorhydrate de bromo-2 (chloro-2 benzyl)-5 tétrahydro-4, 5, 6, 7 thiéno(3, 2-c) pyridine (F : décomposition vers 180° C).

On chauffe à reflux pendant 2 heures, sous atmosphère d'azote, un mélange de base libre (1, 075 g), de magnésium (0, 19 g), de bromure d'isopropyle (0, 57 g) et de tétrahydrofuranne anhydre (10 $cm^3$). Après refroidissement, on ajoute 1, 5 g de perbenzoate de tertiobutyle, agite 15 heures à température ambiante puis une heure à reflux. On verse sur une solution aqueuse de citrate de sodium et de soude et extrait au benzène. Les extraits organiques sont lavés à l'eau, séchés sur sulfate de

sodium et concentrés sous pression réduite. Le résidu huileux est purifié par chromatographie sur une colonne de silice. On récupère 0,25 g d'une huile jaune.

On chauffe à 150°C pendant 9 mn , 100 mg de cette substance et 100 mg d'acide paratoluène sulfonique. Après refroidissement rapide, le milieu est versé sur un mélange tampon phosphate 0,15 M (pH 5,5)-alcool butylique (20 $cm^3$/20 $cm^3$). La phase organique est décantée sous pression réduite. La purification du résidu par chromatographie sur colonne de silice conduit à la (chloro-2 benzyl)-5 tétrahydro-5,6,7,7a 4H-thiéno (3,2-c)pyridinone-2. F = 73 - 74,5°C (éthanol).

Oxalate : F = 168-170°C (éthanol) ; IR(KBr) : ν CO = 1660 $cm^{-1}$ (large).

Chlorhydrate Hémihydrate : F : décomposition vers 180°C (précipitation dans l'acétone).

Base : F = 73-74,5°C (éthanol)

RMN($CDCl_3$) : 7,1 - 7,6 (m,4H) ; 6,2(s,1H) ; 4,2-4,7(m,1H) ; 3,9(s,2H) ; 1,5-4,2(m,6H).

EXEMPLE 2 : Benzyl-5 tétrahydro-5,6,7,7a 4H-thiéno(3,2-c) pyridinone-2, de formule (I)

(R = $C_6H_5$ ; R' = H ; n = 1) dérivé n° 2.

On le prépare selon le mode opératoire de l'exemple 1 à partir de la benzyl-5 tétrahydro-4 5,6,7 thiéno(3,2-c)pyridine.

Maléate : cristaux beiges, F = 132-134°C (isopropanol), IR (KBr) : ν CO : 1680 $cm^{-1}$.

Base : RMN($CDCl_3$) : 7,25 (m,5H) ; 5,90 (s,1H) ; 3,60 (s,2H).

EXEMPLE 3 : (Chloro-4 benzyl)-5 tétrahydro-5,6,7,7a 4H-thiéno (3,2-c)pyridinone-2, de formule (I)

(R = 4-Cl-$C_6H_4$ ; R' = H ; n = 1) dérivé n° 3

On le prépare selon le mode opératoire de l'exemple 1 à partir de (chloro-4 benzyl)-5 tétrahydro-4,5,6,7 thiéno(3,2-c)

pyridine.

Maléate : cristaux beiges, F = 158-160°C (éthanol)

IR(KBr) : ν CO = 1680 $cm^{-1}$

Base : RMN($CDCl_3$) : 7,30(m, 4H) ; 6,0(s, 1H) ; 3,50(s, 2H).

EXEMPLE 4 : (Méthyl-2 benzyl)-5 tétrahydro-5,6,7,7a 4H-thiéno (3,2-c) pyridinone-2, de formule (I)

(R = 2-$CH_3$-$C_6H_4$- ; R' = H ; n = 1) dérivé n° 4

On le prépare selon le mode opératoire de l'exemple 1 à partir de (méthyl-2 benzyl)-5 tétrahydro-4,5,6,7 thiéno (3,2-c)pyridine.

Oxalate : cristaux beiges ; F = 195-197°C (méthanol)

IR (KBr) : ν CO = 1690 $cm^{-1}$

Base : RMN($CDCl_3$) : 7,10(s, 4H) ; 5,90(s, 1H) ; 3,55 (s, 2H) ; 2,30(s, 3H).

EXEMPLE 5 : [(Chloro-2 phényl)-1 éthyl]-5 tétrahydro-5,6,7,7a 4H-thiéno(3,2-c)pyridinone-2, de formule (I)

(R = 2-Cl-$C_6H_4$ ; R' = $CH_3$ ; n = 1) dérivé n° 5

On le prépare selon le mode opératoire de l'exemple 1 à partir de la [(chloro-2 phényl)-1 éthyl]-5 tétrahydro-4,5,6,7 thiéno(3,2-c) pyridine.

Chlorhydrate : cristaux jaunes, F = 140-142°C

IR(KBr) : ν CO = 1690 $cm^{-1}$

Base : RMN($CDCl_3$) : 7,30(m, 4H) ; 6,05 et 5,95(2s, 1H) (2 diastéréoisomères).

EXEMPLE 6 : [(Chloro-2 phényl)-1 propyl]-5 tétrahydro-5,6,7,7a 4H-thiéno(3,2-c)pyridinone-2 de formule (I)

(R = 2-Cl-$C_6H_4$ ; R' = $C_2H_5$ ; n = 1) dérivé n° 6

On le prépare selon le mode opératoire de l'exemple 1 à partir de la [(chloro-2 phényl)-1 propyl]-5 tétrahydro-4,5,6,7 thiéno(3,2-c)pyridine.

Chlorhydrate : cristaux beiges, F = 124-126°C

IR(KBr) : ν CO = 1690 $cm^{-1}$

Base : RMN($CDCl_3$) : 7,30(m,4H) ; 6,05 et 5,90(2s,1H) (2 diastéréoisomères).

EXEMPLE 7 : (cyano-2 benzyl)-5 tétrahydro-5,6,7,7a 4H-thiéno (3,2-c) pyridinone-2, de formule (I)

(R = 2-CN-$C_6H_4$ ; R' = H, n = 1) dérivé n° 7

On le prépare selon le mode opératoire de l'exemple 1 à partir de la (cyano-2 benzyl)-5 tétrahydro-4,5,6,7 thiéno (3,2-c)pyridine.

Oxalate : cristaux beiges, F = 176-178°C (acétonitrile).

IR(KBr) : ν CO : 1700 $cm^{-1}$ ; ν CN : 2210 $cm^{-1}$

Base : RMN($CDCl_3$) : 7,50 (m,4H) ; 6,00(s,1H) ; 3,80(s,2H).

EXEMPLE 8 : (nitro-2 benzyl)-5 tétrahydro-5,6,7,7a 4H-thiéno (3,2-c)pyridinone-2, de formule (I)

(R = 2-$NO_2$-$C_6H_4$ ; R' = H ; n = 1) dérivé n° 8

On le prépare selon le mode opératoire de l'exemple 1 à partir de (nitro-2 benzyl)-5 tétrahydro-4,5,6,7 thiéno(3,2-c) pyridine.

Oxalate : cristaux beiges, F = 186-188°C (isopropanol-éthanol)

IR : νCO = 1685 $cm^{-1}$

Base : RMN($CDCl_3$) : 7,50(m,4H) ; 5,95(s,1H) ; 3,90(s,2H).

EXEMPLE 9 : (bromo-2 benzyl)-5 tétrahydro-5,6,7,7a 4H-thiéno(3,2-c) pyridinone-2, de formule (I)

(R = 2-Br-$C_6H_4$ ; R' = H ; n = 1) dérivé n° 9

On le prépare selon le mode opératoire de l'exemple 1 à partir de (bromo-2 benzyl)-5 tétrahydro-4,5,6,7 thiéno (3,2-c)pyridine.

Oxalate : cristaux beiges, F = 151-153°C (isopropanol).

IR(KBr) : ν CO = 1690 $cm^{-1}$

Base : ($CDCl_3$) : 7,30(m,4H) ; 5,95(s,1H) ; 3,75(s,2H).

Les spectres de RMN du proton ont été réalisés sur appareil Hitachi-Perkin-Elmer R-24A, les déplacements chimiques sont exprimés en ppm, par rapport au TMS pris comme référence interne.

REVENDICATIONS

1.-Procédé de préparation des dérivés de la tétrahydro-5,6,7,7a 4H-thiéno(3,2-c)pyridinone -2 de formule :

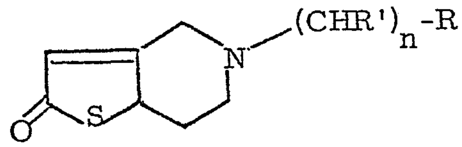

(I)

dans laquelle R représente l'hydrogène ou un radical phényle éventuellement substitué par au moins un atome d'halogène ou un groupe alcoyle inférieur, alcoxy inférieur, nitro, carboxy, alcoxycarbonyle ou cyano; R' représente l'hydrogène, un groupe alcoyle inférieur; et n est zéro ou un nombre entier de 1 à 4, ainsi que leurs sels d'addition avec des acides minéraux ou organiques, caractérisé en ce que :

a) on traite par une solution d'eau oxygénée, un composé de formule II suivante :

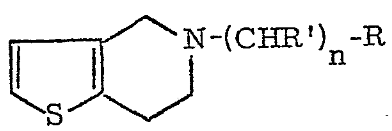

(II),

dans laquelle R, R' et n sont tels que définis ci-dessus, en solution dans un mélange acide acétique/acide bromhydrique;

b) on transforme en organomagnésien, dans un solvant inerte anhydre, le dérivé bromé obtenu à l'issue de l'étape a) et ayant la formule III suivante :

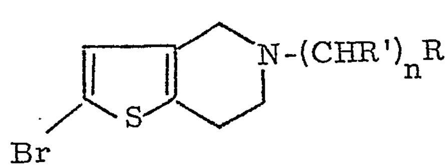

(III) ;

puis on condense le magnésien obtenu avec du perbenzoate de t-butyle pour obtenir le composé de formule IV suivante :

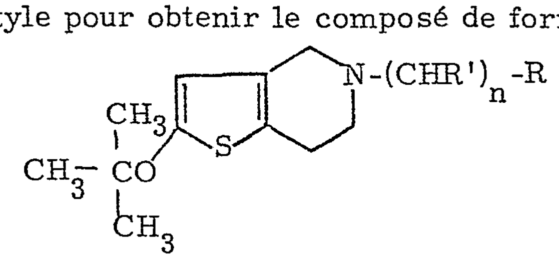

(IV); et

c) on transforme le composé de formule IV obtenu ci-dessus en composé de formule I par chauffage à des températures comprises entre 80 et 180°C, en présence d'un acide minéral ou organique.

2.-Procédé selon la revendication 1, caractérisé en ce que la solution d'eau oxygénée est une solution alcoolique aqueuse telle qu'une solution méthanolique d'eau oxygénée à 30 %.

3.-Procédé selon l'une quelconque des revendications précédentes, caractérisé en ce que le mélange d'acide acétique/acide bromhydrique est compris dans les limites suivantes 50/50 à 80/20.

4.-Procédé selon l'une quelconque des revendications précédentes, caractérisé en ce que le solvant inerte utilisé au cours de l'étape b) est du tétrahydrofuranne anhydre.

5.-Procédé selon l'une quelconque des revendications précédentes, caractérisé en ce que le composé organomagnésien préparé à l'étape b) est obtenu par action de magnésium métallique et d'un halogénure d'alcoyle tel que le bromure d'isopropyle, en milieu anhydre.

6.-Procédé selon l'une quelconque des revendications précédentes, caractérisé en ce que la réaction de condensation avec le perbenzoate de t-butyle est réalisée, au cours de l'étape b), dans le même récipient réactionnel que celui utilisé pour la préparation du magnésien et sans isolement de ce dernier.

7.-Procédé selon l'une quelconque des revendications précédentes, caractérisé en ce que l'acide utilisé au cours de l'étape c) est l'acide p.-toluènesulfonique.

0053950

Numéro de la demande

Office européen des brevets

# RAPPORT DE RECHERCHE EUROPEENNE

EP 81 40 1625

| DOCUMENTS CONSIDERES COMME PERTINENTS | | | CLASSEMENT DE LA DEMANDE (Int. Cl. 3) |
|---|---|---|---|
| Catégorie | Citation du document avec indication, en cas de besoin, des parties pertinentes | Revendication concernée | |
| D | JOURNAL OF THE AMERICAN CHEMICAL SOCIETY, volume 72, décembre 1950, WASHINGTON (US)<br>C.D. HURD et al. "Thienol", pages 5543-5546<br>* en entier *<br>-- | 1 | C 07 D 495/04/<br>A 61 K 31/435<br>(C 07 D 495/041<br>333/00<br>221/00) |
| D | FR - A - 2 215 948 (CENTRE D'ETUDES)<br>----------- | 1 | |
| | | | DOMAINES TECHNIQUES RECHERCHES (Int. Cl. 3) |
| | | | C 07 D 495/04 |
| | | | CATEGORIE DES DOCUMENTS CITES<br>X: particulièrement pertinent à lui seul<br>Y: particulièrement pertinent en combinaison avec un autre document de la même catégorie<br>A: arrière-plan technologique<br>O: divulgation non-écrite<br>P: document intercalaire<br>T: théorie ou principe à la base de l'invention<br>E: document de brevet antérieur, mais publié à la date de dépôt ou après cette date<br>D: cité dans la demande<br>L: cité pour d'autres raisons |
| X | Le présent rapport de recherche a été établi pour toutes les revendications | | &: membre de la même famille, document correspondant |

| Lieu de la recherche | Date d'achèvement de la recherche | Examinateur |
|---|---|---|
| La Haye | 30-01-1982 | ALFARO |

OEB Form 1503.1 06.78